# EUROPEAN PATENT SPECIFICATION

(11) **EP 3 443 346 B1**
(45) Date of publication and mention of the grant of the patent: **30.08.2023**
(21) Application number: 17782960.3
(22) Date of filing: 11.04.2017
(51) Int. Cl.: A61K 9/00, C07K 16/00, A61K 9/08, A61K 9/19, A61K 38/00, A61K 39/00, A61K 47/02, A61K 47/18, A61K 47/26, A61K 39/395

(54) **USE OF AMINO ACIDS AS STABILIZING COMPOUNDS IN PHARMACEUTICAL COMPOSITIONS CONTAINING HIGH CONCENTRATIONS OF PROTEIN-BASED THERAPEUTIC AGENTS**
VERWENDUNG VON AMINOSÄUREN ALS STABILISIERENDE VERBINDUNGEN IN PHARMAZEUTISCHEN ZUSAMMENSETZUNGEN MIT HOHEN KONZENTRATIONEN AN PROTEINBASIERTEN THERAPEUTIKA
UTILISATION D'ACIDES AMINÉS EN TANT QUE COMPOSÉS STABILISANTS DANS DES COMPOSITIONS PHARMACEUTIQUES CONTENANT DES CONCENTRATIONS ÉLEVÉES D'AGENTS THÉRAPEUTIQUES À BASE DE PROTÉINES

(30) Priority: 13.04.2016 US 201662321895 P
(43) Date of publication of application: 20.02.2019
(73) Proprietor: Medimmune, LLC, Gaithersburg, Maryland 20878 (US)
(72) Inventor: PATEL, Sajal, Manubhai, Gaithersburg, MD 20878 (US); CHOUDHARY, Sureshkumar, Bhanaram, Gaithersburg, MD 20878 (US)
(74) Representative: AstraZeneca Intellectual Property
(86) International application number: PCT/US2017/026963
(87) International publication number: WO 2017/180594

(56) References cited:
- WO-A1-2016/034648
- US-A- 5 811 096
- US-A1- 2003 069 183
- US-A1- 2004 022 792
- US-A1- 2005 220 786
- US-A1- 2008 213 282
- US-A1- 2009 291 076
- US-A1- 2010 273 141
- US-A1- 2011 178 019
- US-A1- 2011 262 992
- US-A1- 2012 270 782
- US-A1- 2013 022 625
- US-A1- 2014 127 227
- US-A1- 2014 186 351
- US-A1- 2014 186 361
- US-A1- 2014 186 373
- US-A1- 2014 377 251
- US-A1- 2015 239 970
- US-A1- 2015 283 241
- US-A1- 2015 284 466
- US-B1- 8 613 919

## Description

### Field Of The Invention

The present invention relates to improved pharmaceutical compositions that contain high concentrations of one or more protein biomolecule(s). In particular, the invention relates to such pharmaceutical compositions that include one or more amino acid molecules, particularly arginine, alanine, glycine, lysine or proline, and salts thereof, or mixtures thereof, as stabilizing compounds. The inclusion of such stabilizing compounds decreases reconstitution time whilst improving and/or maintaining the long-term stability of the protein biomolecule, so as to facilitate the treatment, management, amelioration and/or prevention of a disease or condition by the pharmaceutical composition. The invention particularly pertains to such pharmaceutical compositions that lack, or substantially lack, a sugar stabilizing agent.

### Background Of The Invention

Protein-based therapeutic agents (*e.g*., hormones, enzymes, cytokines, vaccines, immunotherapeutics, *etc.)* are becoming increasingly important to the management and treatment of human disease. As of 2014, more than 60 such therapeutics had been approved for marketing, with approximately 140 additional drugs in clinical trial and more than 500 therapeutic peptides in various stages of preclinical development (Fosgerau, K. et al. (2014) "Peptide Therapeutics: Current Status And Future Directions," Drug Discov. Today 20(1):122-128; Kaspar, A.A. et al. (2013) "Future Directions For Peptide Therapeutics Development," Drug Discov. Today 18:807-817).

One impediment to the use of such therapeutics is the physical instability that is often encountered upon their storage (US Patent No. 8,617,576; PCT Publications No. WO 2014/100143 and 2015/061584; Balcão, V.M. et al. (2014) "Structural And Functional Stabilization Of Protein Entities: State-Of-The-Art," Adv. Drug Deliv. Rev. (Epub.): doi: 10.1016/j.addr.2014.10.005; pp. 1-17; Maddux, N.R. et al. (2011) "Multidimensional Methods For The Formulation Of Biopharmaceuticals And Vaccines," J. Pharm. Sci. 100:4171-4197; Wang, W. (1999) "Instability, Stabilization, And Formulation Of Liquid Protein Pharmaceuticals," Int. J. Pharm. 185:129-188; Kristensen, D. et al. (2011) "Vaccine Stabilization: Research, Commercialization, And Potential Impact," Vaccine 29:7122-7124; Kumru, O.S. et al. (2014) "Vaccine Instability In The Cold Chain: Mechanisms, Analysis And Formulation Strategies," Biologicals 42:237-259). Such instability may comprise multiple aspects. A protein-based therapeutic agent may, for example experience operational instability, such as an impaired ability to survive processing operations (*e.g.,* sterilization, lyophilization, cryopreservation, *etc.).* Additionally or alternatively, proteins may experience thermodynamic instability such that a desired secondary or tertiary conformation is lost or altered upon storage. A further, and especially complex problem, lies in the stabilization of therapeutic agents that comprise multimeric protein subunits, with dissociation of the subunits resulting in the inactivation of the product. Kinetic instability is a measure of the capacity of a protein to resist irreversible changes of structure in *in vitro* non-native conditions. Protein aggregation and the formation of inclusion bodies is considered to be the most common manifestation of instability, and is potentially encountered in multiple phases of product development (Wang, W. (2005) "Protein Aggregation And Its Inhibition In Biopharmaceutics," Int. J. Pharm. 289:1-30; Wang, W. (1999) "Instability, Stabilization, And Formulation Of Liquid Protein Pharmaceuticals," Int. J. Pharm. 185:129-188; Arakawa, T. et al. (1993) "Factors Affecting Short-Term And Long-Term Stabilities Of Proteins," Adv. Drug Deliv. Rev. 10:1-28; Arakawa, T. et al. (2001) "Factors Affecting Short-Term And Long-Term Stabilities Of Proteins," Adv. Drug Deliv. Rev. 46:307-326). Such issues of instability can affect not only the efficacy of the therapeutic but its immunogenicity to the recipient patient. Protein instability is thus one of the major drawbacks that hinders the use of protein-based therapeutic agent (Balcão, V.M. et al. (2014) "Structural And Functional Stabilization Of Protein Entities: State-Of The-Art," Adv. Drug Deliv. Rev. (Epub.): doi: 10.1016/j.addr.2014.10.005; pp. 1-17).

Stabilization of protein-based therapeutic agents entails preserving the structure and functionality of such agents, and has been accomplished by establishing a thermodynamic equilibrium between such agents and their (micro)environment (Balcão, V.M. et al. (2014) "Structural And Functional Stabilization Of Protein Entities: State-Of The-Art," Adv. Drug Deliv. Rev. (Epub.): doi: 10.1016/j.addr.2014.10.005; pp. 1-17). One approach to stabilizing protein-based therapeutic agents involves altering the protein to contain additional covalent *(e.g.,* disulfide) bonds so as to increase the enthalpy associated with a desired conformation. Alternatively, the protein may be modified to contain additional polar groups so as to increase its hydrogen bonding with solvating water molecules (Mozhaev, V.V. et al. (1990) "Structure-Stability Relationships In Proteins: A Guide To Approaches To Stabilizing Enzymes," Adv. Drug Deliv. Rev. 4:387-419; Iyer, P.V. et al. (2008) "Enzyme Stability And Stabilization - Aqueous And Non-Aqueous Environment," Process Biochem. 43:1019-1032).

A second approach to stabilizing protein-based therapeutic agents involves reducing the chemical activity of the water present in the protein's microenvironment, for example by freezing the water, adding specific solutes, or lyophilizing the pharmaceutical composition (see, *e.g.,* Castronuovo, G. (1991) "Proteins In Aqueous Solutions. Calorimetric Studies And Thermodynamic Characterization," Thermochim. Acta 193:363-390).

Employed solutes range from small molecular weight ions (e.g., salts, buffering agents) to intermediate sized solutes (e.g., amino acids, sugars) to larger molecular weight compounds (e.g., polymers, proteins) (Kamerzell, T.J. et al. (2011) "Protein-Excipient Interactions: Mechanisms And Biophysical Characterization Applied To Protein Formulation Development," Adv. Drug Deliv. Rev. 63:1118-1159).

For example, such solutes have included budesonide, dextran DMSO glycerol, glucose, inulin, lactose, maltose, mannitol, PEG, piroxicam, PLGA, PVA sorbitol, sucrose, trehalose and urea (Ohtake, S. et al. (2011) "Trehalose: Current Use and Future Applications," J. Pharm. Sci. 100(6):2020-2053; Willart, J.P. et al. (2008) "Solid State Amorphization of Pharmaceuticals," Molec. Pharmaceut. 5(6):905-920; Kumru, O.S. et al. (2014) "Vaccine Instability In The Cold Chain: Mechanisms, Analysis And Formulation Strategies," Biologicals 42:237-259; Somero, G.N. (1995) "Proteins And Temperature," Annu. Rev. Physiol. 57: 43-68; Sasahara, K. et al. (2003) "Effect Of Dextran On Protein Stability And Conformation Attributed To Macromolecular Crowding," J. Mol. Biol. 326:1227-1237; Jain, N.K. et al. (2014) "Formulation And Stabilization Of Recombinant Protein Based Virus-Like Particle Vaccines," Adv. Drug Deliv. Rev. (Epub.) doi: 10.1016/j.addr.2014.10.023; pp. 1-14; Kissmann, J. et al. (2011) "HINI Influenza Virus-Like Particles: Physical Degradation Pathways And Identification Of Stabilizers," J. Pharm. Sci. 100:634-645; Kamerzell, T.J. et al. (2011) "Protein-Excipient Interactions: Mechanisms And Biophysical Characterization Applied To Protein Formulation Development," Adv. Drug Deliv. Rev. 63:1118-1159).

Sugars such as sucrose and trehalose dihydrate are typically used as lyoprotectants and cryoprotectants in lyophilized therapeutic protein formulations to improve drug product stability, *e.g.,* for storage at 2-8 °C (US Patents No. 8,617,576 and 8,754,195). Trehalose, in particular, has been widely used as a stabilizing agent; it is used in a variety of research applications and is contained in several commercially available therapeutic products, including HERCEPTIN^{®}, AVASTIN^{®}, LUCENTIS^{®}, and ADVATE^{®} (Ohtake, S. et al. (2011) "Trehalose: Current Use and Future Applications," J. Pharm. Sci. 100(6):2020-2053).

The amino acids histidine, arginine, glutamate, glycine, proline, lysine and methionine have been mentioned as natural compounds that stabilize proteins. Human serum albumin (HSA) and gelatin have been mentioned as being protein stabilizers (US Patent No. 8,617,576; US Patent Publication No. 2015/0118249; Kamerzell, T.J. et al. (2011) "Protein-Excipient Interactions: Mechanisms And Biophysical Characterization Applied To Protein Formulation Development," Adv. Drug Deliv. Rev. 63:1118-1159; Kumru, O.S. et al. (2014) "Vaccine Instability In The Cold Chain: Mechanisms, Analysis And Formulation Strategies," Biologicals 42:237-259; Arakawa, T. et al. (2007) "Suppression Of Protein Interactions By Arginine: A Proposed Mechanism Of The Arginine Effects," Biophys. Chem. 127:1-8; Arakawa, T. et al. (2007) "Biotechnology Applications Of Amino Acids In Protein Purification And Formulations," Amino Acids 33:587-605; Chen, B. (2003) "Influence Of Histidine On The Stability And Physical Properties Of A Fully Human Antibody In Aqueous And Solid Forms," Pharm. Res. 20:1952-1960; Tian, F. et al. (2007) "Spectroscopic Evaluation Of The Stabilization Of Humanized Monoclonal Antibodies In Amino Acid Formulations," Int. J. Pharm. 335:20-31; Wade, A.M. et al. (1998) "Antioxidant Characteristics Of L-Histidine," J. Nutr. Biochem. 9:308-315; Yates, Z. et al. (2010) "Histidine Residue Mediates Radical-Induced Hinge Cleavage Of Human Iggl," J. Biol. Chem. 285:18662-18671; Lange, C. et al. (2009) "Suppression Of Protein Aggregation By L-Arginine," Curr. Pharm. Biotechnol. 10:408-414; Nakakido, M. et al. (2009) "To Be Excluded Or To Bind, That Is The Question: Arginine Effects On Proteins," Curr. Pharm. Biotechnol. 10:415-420; Shukla, D. et al. (2010) "Interaction Of Arginine With Proteins And The Mechanism By Which It Inhibits Aggregation," J. Phys. Chem. B 114:13426-13438; Pyne, A. et al. (2001) "Phase Transitions Of Glycine In Frozen Aqueous Solutions And During Freeze-Drying," Pharm. Res. 18:1448-1454; Lam, X.M. et al. (1997) "Antioxidants For Prevention Of Methionine Oxidation In Recombinant Monoclonal Antibody HER2," J. Pharm. Sci. 86:1250-1255; Maeder, W. et al. (2011) "Local Tolerance And Stability Up To 24 Months Of A New 20% Proline-Stabilized Polyclonal Immunoglobulin For Subcutaneous Administration," Biologicals 39:43-49; Kadoya, S. et al. (2010) "Freeze-Drying Of Proteins With Glass-Forming Oligosaccharide-Derived Sugar Alcohols," Int. J. Pharm. 389:107-113; Golovanov, A.P. et al. (2004) "A Simple Method For Improving Protein Solubility And Long-Term Stability, J. Am. Chem. Soc. 126:8933-8939).

Typically, a protein-to-stabilizer compound ratio of 1:1 or 1:2 (w/w) has been used to achieve optimal stability for lower protein concentrations (< 50 mg/mL). However, for higher protein concentrations (≥ 50 mg/mL), protein-to-stabilizer compound ratios in the 1:1 or 1:2 (w/w) range are less desirable. For example, such high sugar concentrations can result in high viscosity, which impose challenges during fill-finish operations and in drug-delivery and can require increased reconstitution times for lyophilized formulations. Moreover, the reconstituted formulations can exhibit high osmolality, far outside the desired isotonic range, especially if partial reconstitution is desired in order to achieve a higher protein concentration. Finally, high concentration protein formulations with protein-to-stabilizer compound ratios in the 1:1 or 1:2 (w/w) range can exhibit thermal characteristics that require unacceptably long lyophilization process times at much lower temperatures.

The need to reconstitute such protein-based therapeutic agents imposes a second impediment to their use. Factors that govern reconstitution time remain poorly understood (Beech, K.E. et al. (2015) "Insights Into The Influence Of The Cooling Profile On The Reconstitution Times Of Amorphous Lyophilized Protein Formulations," Eur. J. Pharmaceut. Biopharmaceut. 96:247-254). The time needed to achieve full reconstitution of conventional compositions may be significant (*e.g.,* 20-40 minutes or more), and products that have not been fully reconstituted may be detrimental to recipient patients. Additionally, the reconstitution procedure can differ depending on the product, which can add further complexity to the administration process. For example, after the addition of a diluent, a product may require swirling at set intervals, or may require being left undisturbed, in order to achieve complete reconstitution (Beech, K.E. et al. (2015) "Insights Into The Influence Of The Cooling Profile On The Reconstitution Times Of Amorphous Lyophilized Protein Formulations," Eur. J. Pharmaceut. Biopharmaceut. 96:247-254).

Further compositions comprising a protein biomolecule and stabilizing amino acids are disclosed in US 2009/291076 A1, US 2003/069183 A1, US 2012/270782 A1, US 2015/239970 A1, WO 2016/034648 A1, US 2008/213282 A1, US 2014/186361 A1, US 2014/186373 A1, US 2014/377251 A1, US 2011/178019 A1, US 2015/283241 A1, US 8 613 919 B1, US 2005/220786 A1, US 5 811 096 A, US 2014/127227 A1.

Thus, despite all of such advances, a need remains for formulations suitable for stabilizing protein-based pharmaceutical compositions, particularly without a sugar stabilizing agent, such that the pharmaceutical compositions would exhibit improved viscosity and reconstitution times and enhanced stability, both in lyophilized / cryopreserved form and following reconstitution. The present invention is directed to this and other goals.

### Summary Of The Invention

The present invention relates to improved pharmaceutical compositions that contain high concentrations of one or more protein biomolecule(s). In particular, the invention relates to such pharmaceutical compositions that include one or more amino acid molecules, particularly arginine, alanine, glycine, lysine or proline, or derivatives and salts thereof, or mixtures thereof, as stabilizing compounds. The inclusion of such stabilizing compounds decreases reconstitution time whilst improving and/or maintaining the long-term stability of the protein biomolecule, so as to facilitate the treatment, management, amelioration and/or prevention of a disease or condition by the pharmaceutical composition. The invention particularly pertains to such pharmaceutical compositions that lack, or substantially lack, a sugar stabilizing agent.

In detail, the invention concerns a pharmaceutical composition according to claim 1.

The invention further concerns the embodiment of the above-indicated pharmaceutical composition wherein the composition preferably completely lacks a sugar stabilizing compound.

The invention further concerns the embodiment of all of the above-indicated pharmaceutical compositions wherein the protein biomolecule is an antibody or an antibody-based immunotherapeutic.

The invention further concerns the embodiment of the above-indicated pharmaceutical compositions wherein the protein biomolecule is an antibody or an antibody-based immunotherapeutic, and the antibody is selected from the antibodies of Table 1.

The invention further concerns the embodiment of any of the above-indicated pharmaceutical compositions wherein the composition preferably comprises at least two protein biomolecules.

The invention further concerns the embodiments of any of the above-indicated pharmaceutical compositions wherein the stabilizing compound is 5% w/v arginine or 4% w/ v alanine with 2% w/v arginine, according to claim 1.

The invention further concerns the embodiments of any of the above-indicated pharmaceutical compositions wherein the stabilizing compound may further comprise glycine according to claim 1.

The invention further concerns the embodiment of any of the above-indicated pharmaceutical compositions wherein the composition preferably comprises at least two stabilizing compounds.

The invention further concerns the embodiment of any of the above-indicated pharmaceutical compositions wherein the pH of the pharmaceutical composition is 6.

The invention further concerns the embodiment of any of the above-indicated pharmaceutical compositions wherein the buffer is present at 25mM.

The invention further concerns the embodiments of any of the above-indicated pharmaceutical compositions wherein the buffer is histidine / histidine-HCl.

The invention further concerns the embodiment of any of the above-indicated pharmaceutical compositions wherein the pharmaceutical composition additionally comprises a non-ionic detergent polysorbate-80 (PS-80) at a concentration of 0.02% (w/v).

According to the invention, the above-indicated pharmaceutical compositions are in a lyophilisate form.

The invention further concerns the embodiment of any of the above-indicated pharmaceutical compositions wherein the presence of the stabilizing compound(s) causes the reconstitution time of a lyophilisate of the pharmaceutical composition to be less than 20 minutes, less than 15 minutes, less than 10 minutes, less than 8 minutes, less than 5 minutes, or less than 2 minutes.

The invention further concerns the embodiment of any of the above-indicated pharmaceutical compositions wherein the presence of the stabilizing compound(s) preferably enhances a stability characteristic of the pharmaceutical composition by more than 400%, by more than 200%, by more than 100%, by more than 50%, or by more than 10%, relative to such stability characteristic as observed in the complete absence of the amino acid stabilizing compound(s).

The invention further concerns the embodiment of any of the above-indicated pharmaceutical compositions wherein the presence of the stabilizing compound(s) enhances a stability characteristic of the pharmaceutical composition by more than 50%, by more than 20%, by more than 10%, by more than 5%, or by more than 1%, relative to such stability characteristic as observed in the complete absence of a sugar stabilizing compound.

The invention further concerns an ampoule, vial, cartridge, syringe or sachette that contains any of the above-indicated pharmaceutical compositions.

The invention further concerns of the above-indicated pharmaceutical compositions for use as a medicament.

The invention further concerns a use of one or more amino acids, such as arginine, alanine, glycine, lysine or proline, as a replacement of one or more sugars in a pharmaceutical formulation to decrease reconstitution time.

### Brief Description Of The Drawings

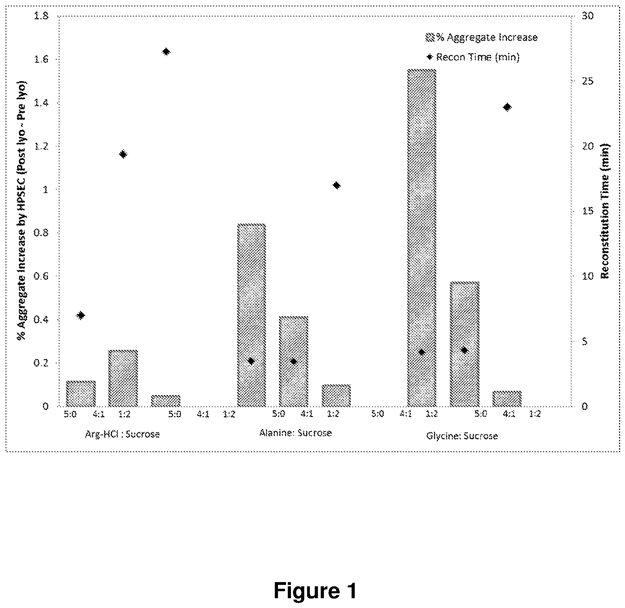

**Figure 1** shows the observed reconstitution times and degree of aggregation (assessed by high performance size-exclusion chromatography (HPSEC)) of a lyophilized formulation of a pharmaceutical composition containing a high concentration (100 mg/mL) of an exemplary protein biomolecule (a human IgG1 monoclonal antibody) with various amino acid-sugar combinations. Percent aggregate increase post-lyophilization (left axis) is shown as bars, reconstitution times (right axis) are shown as diamonds.

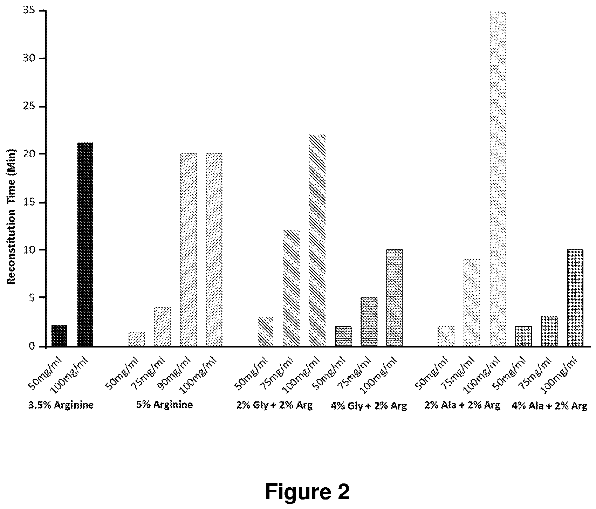

**Figure 2** shows the effects of protein concentration and amino acid excipients on reconstitution times for the indicated lyophilized formulations of pharmaceutical compositions containing a high concentration (50 mg/mL, 75 mg/mL or 100 mg/mL) of a human IgG1 monoclonal antibody. The human IgG1 monoclonal antibody was employed as an exemplary protein biomolecule.

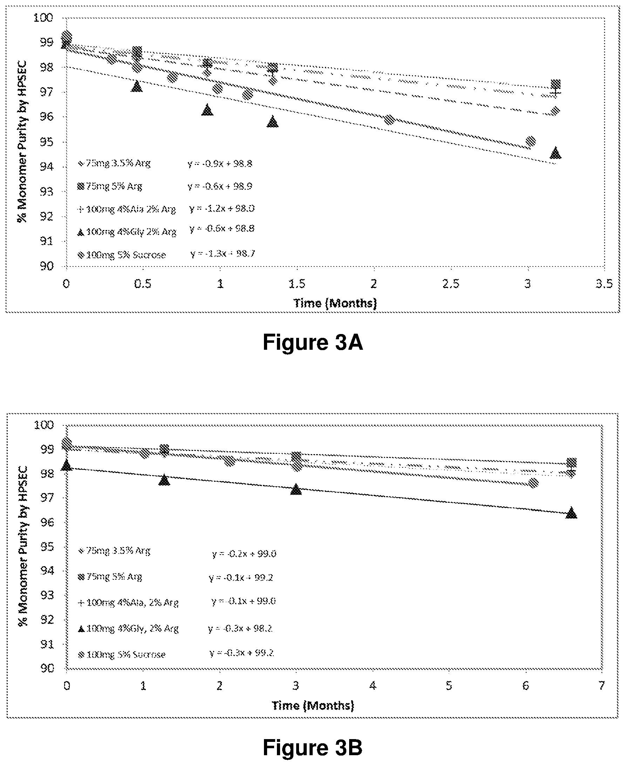

**Figures 3A-3B** show the percent monomer purity over time, as determined by HPSEC for formulations of pharmaceutical compositions containing 75 mg/mL or 100 mg/mL of a human IgG1 monoclonal antibody with various added excipients as shown, at 40 °C, 60% relative humidity (**Figure 3A**) and at 25 °C, 75% relative humidity (**Figure 3B**). The human IgG1 monoclonal antibody was employed as an exemplary protein biomolecule.

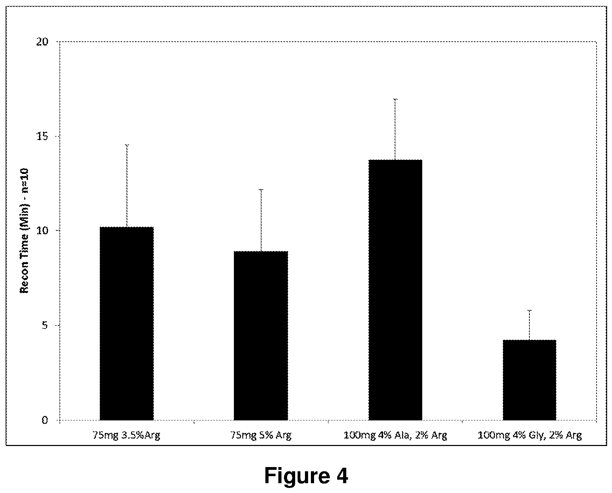

**Figure 4** shows the reconstitution times for lyophilized formulations, as shown, of pharmaceutical compositions containing a high concentration (75 mg/mL or 100 mg/mL) of a human IgG1 monoclonal antibody, which was employed as an exemplary protein biomolecule. The results are the averages of 10 replicate experiments.

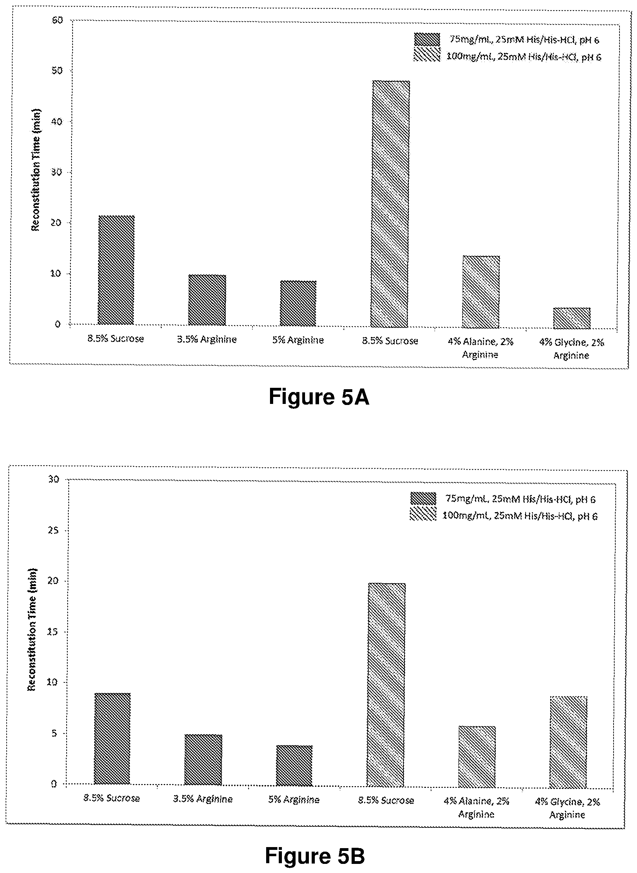

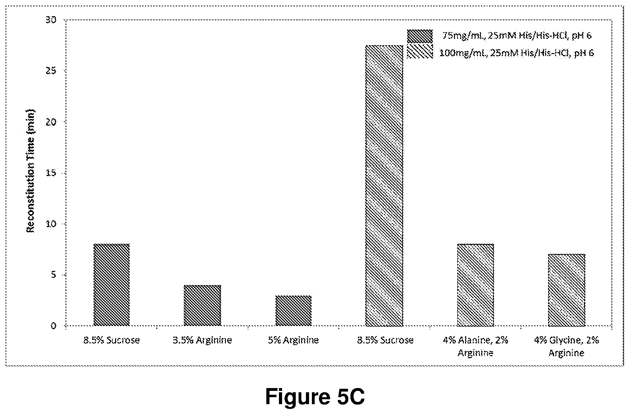

**Figures 5A-5C** show reconstitution times of lyophilized formulations prepared with 75 mg/mL or 100 mg/mL of one of three different exemplary proteins with various amino acid excipients. **Figure 5A** shows reconstitution times for a human IgG1 monoclonal antibody. **Figure 5B** shows reconstitution times for a Tn3-HSA fusion protein. **Figure 5C** shows reconstitution times for a humanized IgG4 monoclonal antibody.

### Detailed Description Of The Invention

The present invention relates to improved pharmaceutical compositions that contain high concentrations of one or more protein biomolecule(s). In particular, the invention relates to such pharmaceutical compositions that include one or more amino acid molecules, particularly arginine, alanine, glycine, lysine or proline, and salts thereof, or mixtures thereof, as stabilizing compounds, according to claim 1. The inclusion of such stabilizing compounds decreases reconstitution time whilst improving and/or maintaining the long-term stability of the protein biomolecule, so as to facilitate the treatment, management, amelioration and/or prevention of a disease or condition by the pharmaceutical composition. The invention particularly pertains to such pharmaceutical compositions that lack, or substantially lack, a sugar stabilizing agent.

### I. The Pharmaceutical Compositions of the Present Invention

As used herein, the term "pharmaceutical composition" is intended to refer to a "therapeutic" medicament (*i.e*., a medicament formulated to treat an existing disease or condition of a recipient subject) or a "prophylactic" medicament (*i*.*e.,* a medicament formulated to prevent or ameliorate the symptoms of a potential or threatened disease or condition of a recipient subject) containing one or more protein biomolecules as its active therapeutic or prophylactic agent or component. The pharmaceutical compositions of the present invention comprise one or more protein biomolecule(s) that serve(s) as an active agent or component of the composition. For therapeutic use, the pharmaceutical composition will contain and provide a "therapeutically effective" amount of the protein biomolecule(s), which is an amount that reduces or ameliorates the progression, severity, and/or duration of a disease or condition, and/or ameliorates one or more symptoms associated with such disease or condition. For prophylactic use, the pharmaceutical composition will contain and provide a "prophylactically effective" amount of the protein biomolecule(s), which is an amount that is sufficient to result in the prevention of the development, recurrence, onset or progression of a disease or condition. The recipient subject is an animal, preferably a mammal including a non-primate *(e.g.,* a cow, pig, horse, cat, dog, rat, or mouse), or a primate (*e.g.,* a chimpanzee, a monkey such as a cynomolgus monkey, and a human), and is more preferably a human.

### II. The Stabilizing Compounds of the Pharmaceutical Compositions of the Present Invention

The stabilizing compounds of the present invention are "lyoprotectants" (and as such serve to protect the protein biomolecule of the pharmaceutical composition from denaturation during freeze-drying and subsequent storage) and/or "cryoprotectants" (and as such serve to protect the protein biomolecule of the pharmaceutical composition from denaturation caused by freezing). A "stabilizing" compound is said to "stabilize" or "protect" a protein biomolecule of a pharmaceutical compositions of the present invention, if it serves to preserve the structure and functionality of the protein biomolecule that is the active agent or component of the composition, relative to changes in such structure and functionality observed in the absence of such formulation. A stabilizing compound is one that serves to prevent or decrease the extent of freezing or melting of a composition at that composition's normal melt temperature (Tm).

The "protection" provided to the protein biomolecule may be assessed using high performance size-exclusion chromatography ("HPSEC"), which is an industry standard technique for the detection and quantification of pharmaceutical protein aggregates (US Patent Publication No. 2015/0005475; Gabrielson, J.P. et al. (2006) "Quantitation Of Aggregate Levels In A Recombinant Humanized Monoclonal Antibody Formulation By Size-Exclusion Chromatography, Asymmetrical Flow Field Flow Fractionation, And Sedimentation Velocity," J. Pharm. Sci. 96(2):268-279; Liu, H. et al. (2009) "Analysis Of Reduced Monoclonal Antibodies Using Size Exclusion Chromatography Coupled With Mass Spectrometry," J. Amer. Soc. Mass Spectrom. 20:2258-2264; Mahler, H. C. et al. (2008) "Protein Aggregation: Pathways, Induction Factors And Analysis," J. Pharm. Sci. 98(9):2909-2934). Such protection permits the protein biomolecule to exhibit "low to undetectable levels" of fragmentation (*i.e.,* such that, in a sample of the pharmaceutical composition, more than 80%, 85%, 90% 95%, 98%, or 99% of the protein biomolecule migrates in a single peak as determined by HPSEC and/or "low to undetectable levels" of loss of the biological activity/ies associated (*i.e*., such that, in a sample of the pharmaceutical composition, more than 80%, 85%, 90% 95%, 98%, or 99% of the protein biomolecule present exhibits its initial biological activity/ies as measured by HPSEC, and/or low to undetectable levels" of aggregation (*i.e.,* such that, in a sample of the pharmaceutical composition, no more than 5%, no more than 4%, no more than 3%, no more than 2%, no more than 1%, and most preferably no more than 0.5%, aggregation by weight protein as measured by HPSEC. The "long-term" stability provided by the pharmaceutical compositions of the present permit such compositions to be stored for more than 3 months, more than 6 months, more than 9 months, more than 1 year, more than 18 months, more than 2 years, or more than 30 months.

The "stabilizing compounds" of the present invention achieve shorter reconstitution times for lyophilized pharmaceutical compositions that contain high concentrations of one or more protein biomolecule(s). According to the invention, said stabilizing compounds are disclosed in claim 1. Such amino acid molecules will preferably be L-amino acid molecules, but may be D-amino acid molecules or any combination of D- and L-amino acid molecules, including a racemic mixture thereof. Preferably, the presence of such stabilizing compound(s) of the present invention will be sufficient to cause the reconstitution time of a lyophilisate of the pharmaceutical composition to be less than 20 mins, less than 15 mins, less than 10 mins, less than 8 mins, less than 5 mins, or less than 2 mins, and to enhance a stability characteristic (*e.g.,* a lyoprotective or cryoprotective property, such as single dosage reconstitution time, mean shelf life, percent activity remaining at a designated time interval at a set temperature (*e.g.,* a subzero temperature, room temperature or an elevated temperature), *etc.*) of the pharmaceutical composition by more than 400%, by more than 200%, by more than 100%, by more than 50%, or by more than 10%, relative to such stability characteristic as observed in the complete absence of amino acid stabilizing compound(s).

With respect to such amino acids molecules, the term "derivatives and salts thereof" denotes any pharmaceutically acceptable salt or amino acid derivative, such as those disclosed in REMINGTON: THE SCIENCE AND PRACTICE OF PHARMACY, 21th Edition, Gennaro, Ed., Mack Publishing Co., Easton, PA, 2005. Such derivatives include substituted amines, amino alcohols, aldehydes, lactones, esters, hydrates, *etc.* Exemplary derivatives of alanine include: 2-allyl-glycine, 2-aminobutyric acid, cis-amiclenomycin, adamanthane, *etc.* Exemplary derivatives of arginine include: 2-amino-3-guanidinopropionic acid, 2-amino-4-guanidinobutryric acid, 5-methyl-arginine, arginine methyl ester, arginine-O-tBu, canavanine, citrulline, c-γ-hydroxy arginine, homoarginine, N-tosyl-arginine, N_{ω}-nitro-arginine, thio-citrulline, *etc.* Exemplary derivatives of lysine include: diaminobutyric acid, 2,3-diaminopropanoic acid, (2s)-2,8-diaminoactanoic acid, ornithine, thialysine, *etc.* Exemplary derivatives of proline include: trans-1-acetyl-4-hydroxyproline, 3,4-dehydroproline, cis-3-hydroxyproline, cis-4-hydroxyproline, trans-3-hydroxyproline, trans-4-hydroxyproline, α-methylproline, pipecolic acid, *etc.*

Salts of such amino acids molecules and their derivatives include addition salts of such molecules such as those derived from an appropriate acid, *e.g*., hydrochloric, sulphuric, phosphoric, maleic, fumaric, citric, tartaric, lactic, acetic or p-toluenesulphonic acid. Particularly preferred are hydrochloride salts.

Such stabilizing compounds can be used individually or in combination in the pharmaceutical compositions of the present invention (*e.g*., any two stabilizing compounds, any three stabilizing compounds, any four stabilizing compounds, any five stabilizing compounds, or any combination of more than five of such stabilizing compounds, according to claim 1.

As discussed above, sugars such as dextran, sucrose, trehalose dihydrate are typically used as stabilizing compounds in lyophilized therapeutic protein formulations. In a highly preferred embodiment of the present invention, the pharmaceutical compositions of the present invention will substantially lack (*i.e.,* be substantially free of) a sugar stabilizing compound, and in a more highly preferred embodiment of the present invention, the pharmaceutical compositions of the present invention will completely lack (*i.e.,* be completely free of) a sugar stabilizing compound. As used herein, a pharmaceutical composition of the present invention is said to "substantially lack sugar stabilizing compound(s)" if the presence of such compounds does not enhance a stability characteristic (*e.g.,* a lyoprotective or cryoprotective property) of the pharmaceutical composition by more than 50%, by more than 20%, by more than 10%, by more than 5%, or by more than 1%, relative to such stability characteristic as observed in the complete absence of such sugar stabilizing compound(s). As used herein, a pharmaceutical composition of the present invention is said to "completely lack sugar stabilizing compound(s)" if the presence of such compound(s) is not detectable. It is preferred that the pharmaceutical compositions of the present invention will completely lack any sugar stabilizing compound.

Sugars such as sucrose and trehalose dihydrate are typically used as excipients in lyophilized therapeutic protein formulations to improve drug product stability, *e.g.,* for storage at 2-8 °C (US Patents No. 8,617,576 and 8,754,195). Trehalose, in particular, has been widely used as a stabilizing agent; it is used in a variety of research applications and is contained in several commercially available therapeutic products, including HERCEPTIN^{®}, AVASTIN^{®}, LUCENTIS^{®}, and ADVATE^{®} (Ohtake, S. et al. (2011) "Trehalose: Current Use and Future Applications," J. Pharm. Sci. 100(6):2020-2053).

### III. The Protein Biomolecules Of The Pharmaceutical Compositions Of The Present Invention

The stabilizing compounds of the present invention are particularly suitable for use in pharmaceutical compositions that contain high concentrations of one or more protein biomolecule(s) as their active agents or components. As used herein, the term "high concentration" denotes a concentration of the protein biomolecule(s) that is 75 mg/mL or 100 mg/mL, according to claim 1.

Without limitation, the "protein biomolecules" contained in such pharmaceutical compositions may be any kind of protein molecule, including single polypeptide chain proteins or multiple polypeptide chain proteins. As used herein the term protein biomolecule does not connote that the molecule is of any particular size and is intended to include protein biomolecules that comprise fewer than 5, fewer than 10, fewer than 20 fewer than 30, fewer than 40 or fewer than 50 amino acid residues, as well as protein biomolecules that comprise more than 50, more than 100, more than 200 more than 300, more than 400, or more than 500 amino acid residues.

Examples of protein biomolecules that must be present in the pharmaceutical compositions of the present invention are provided in Tables 1 and 2, and include antibody or antibody-based immunotherapeutics (for example, palivizumab which is directed to an epitope in the A antigenic site of the F protein of respiratory syncytial virus (RSV) (SYNAGIS^{®}; US Patents No. 8,460,663 and 8,986,686), antibody directed against angiopoietin-2 (US Patents No. 8,507,656 and 8,834,880); antibody directed against Delta-like Protein Precursor 4 (DLL4) (US Patent No. 8,663,636; US Patent Publication No. 2015/0005475; PCT Publication No. WO 2013/113898); antibody directed against Platelet-Derived Growth Factor-α (PDGRF-α) (US Patent No. 8,697,664); antibody directed against alpha-V-beta-6 integrin (αVβ6) (US Patent No. 8,894,998; antibody directed against Growth and Differentiation Factor (GDF-8) (US Patent No. 8,697,664).

| **Table 1 Antibody and Immunotherapeutic Molecules** | | |
|---|---|---|
| **Antibody Name** | **Disease-Associated Antigen** | **Therapeutic Target Application** |
| 3F8 | Gd2 | Neuroblastoma |
| 8H9 | B7-H3 | Neuroblastoma, Sarcoma, Metastatic Brain Cancers |
| Abagovomab | CA-125 | Ovarian Cancer |
| Abciximab | CD41 | Platelet Aggregation Inhibitor |
| Actoxumab | *Clostridium Difficile* | *Clostridium Difficile* Infection |
| Adalimumab | TNF-A | Rheumatoid Arthritis, Crohn's Disease, Plaque Psoriasis, Psoriatic Arthritis, Ankylosing Spondylitis, Juvenile Idiopathic Arthritis, Hemolytic Disease Of The Newborn |
| Adecatumumab | Epcam | Prostate And Breast Cancer |
| Aducanumab | Beta-Amyloid | Alzheimer's Disease |
| Afelimomab | TNF-A | Sepsis |
| Afutuzumab | CD20 | Lymphoma |
| Alacizumab | VEGFR2 | Cancer |
| Ald518 | Il-6 | Rheumatoid Arthritis |
| Alemtuzumab | CD52 | Multiple Sclerosis |
| Alirocumab | NARP-1 | Hypercholesterolemia |
| Altumomab | CEA | Colorectal Cancer |
| Amatuximab | Mesothelin | Cancer |
| Anatumomab Mafenatox | TAG-72 | Non-Small Cell Lung Carcinoma |
| Anifrolumab | Interferon A/B Receptor | Systemic Lupus Erythematosus |
| Anrukinzumab | IL-13 | Cancer |
| Apolizumab | HLA-DR | Hematological Cancers |
| Arcitumomab | CEA | Gastrointestinal Cancer |
| Aselizumab | L-Selectin (CD62L) | Severely Injured Patients |
| Atinumab | RTN4 | Cancer |
| Atlizumab | IL-6 Receptor | Rheumatoid Arthritis |
| Atorolimumab | Rhesus Factor | Hemolytic Disease Of The Newborn |
| Bapineuzumab | Beta-Amyloid | Alzheimer's Disease |
| Basiliximab | CD25 | Prevention Of Organ Transplant Rejections |
| Bavituximab | Phosphatidylserine | Cancer, Viral Infections |
| Bectumomab | CD22 | Non-Hodgkin's Lymphoma (Detection) |
| Belimumab | BAFF | Non-Hodgkin Lymphoma |
| Benralizumab | CD125 | Asthma |
| Bertilimumab | CCL11 (Eotaxin-1) | Severe Allergic Disorders |
| Besilesomab | CEA-Related Antigen | Inflammatory Lesions And Metastases (Detection) |
| Bevacizumab | VEGF-A | Metastatic Cancer, Retinopathy Of Prematurity |
| Bezlotoxumab | *Clostridium difficile* | *Clostridium difficile* Infection |
| Biciromab | Fibrin II, Beta Chain | Thromboembolism (Diagnosis) |
| Bimagrumab | ACVR2B | Myostatin Inhibitor |
| Bivatuzumab | CD44 V6 | Squamous Cell Carcinoma |
| Blinatumomab | CD19 | Cancer |
| Blosozumab | SOST | Osteoporosis |
| Brentuximab | CD30 (TNFRSF8) | Hematologic Cancers |
| Briakinumab | IL-12, IL-23 | Psoriasis, Rheumatoid Arthritis, Inflammatory Bowel Diseases, Multiple Sclerosis |
| Brodalumab | IL-17 | Inflammatory Diseases |
| Canakinumab | IL-1 | Rheumatoid Arthritis |
| Cantuzumab | MUC1 | Cancers |
| Cantuzumab Mertansine | Mucin Canag | Colorectal Cancer |
| Caplacizumab | VWF | Cancers |
| Capromab | Prostatic Carcinoma Cells | Prostate Cancer (Detection) |
| Carlumab | MCP-1 | Oncology/Immune Indications |
| Catumaxomab | Epcam, CD3 | Ovarian Cancer, Malignant Ascites, Gastric Cancer |
| Cc49 | Tag-72 | Tumor Detection |
| Certolizumab | TNF-A | Crohn's Disease |
| Cetuximab | EGFR | Metastatic Colorectal Cancer And Head And Neck Cancer |
| Ch.14.18 | Undetermined | Neuroblastoma |
| Citatuzumab | Epcam | Ovarian Cancer And Other Solid Tumors |
| Cixutumumab | IGF-1 Receptor | Solid Tumors |
| Clazakizumab | *Oryctolagus Cuniculus* | Rheumatoid Arthritis |
| Clivatuzumab | MUC1 | Pancreatic Cancer |
| Conatumumab | TRAIL-R2 | Cancer |
| Concizumab | TFPI | Bleeding |
| Cr6261 | Influenza A Hemagglutinin | Infectious Disease/Influenza A |
| Crenezumab | 1-40-B-Amyloid | Alzheimer's Disease |
| Dacetuzumab | CD40 | Hematologic Cancers |
| Daclizumab | CD25 | Prevention Of Organ Transplant Rejections |
| Dalotuzumab | Insulin-Like Growth Factor I Receptor | Cancer |
| Daratumumab | CD38 | Cancer |
| Demcizumab | DLL4 | Cancer |
| Denosumab | RANKL | Osteoporosis, Bone Metastases |
| Detumomab | B-Lymphoma Cell | Lymphoma |
| Dorlimomab Aritox | Undetermined | Cancer |
| Drozitumab | DR5 | Cancer |
| Duligotumab | HER3 | Cancer |
| Dupilumab | IL4 | Atopic Diseases |
| Dusigitumab | ILGF2 | Cancer |
| Ecromeximab | GD3 Ganglioside | Malignant Melanoma |
| Eculizumab | C5 | Paroxysmal Nocturnal Hemoglobinuria |
| Edobacomab | Endotoxin | Sepsis Caused By Gram-Negative Bacteria |
| Edrecolomab | Epcam | Colorectal Carcinoma |
| Efalizumab | LFA-1 (CD11a) | Psoriasis (Blocks T Cell Migration) |
| Efungumab | Hsp90 | Invasive Candida Infection |
| Eldelumab | Interferon-Gamma-Induced Protein | Crohn's Disease, Ulcerative Colitis |
| Elotuzumab | SLAMF7 | Multiple Myeloma |
| Elsilimomab | IL-6 | Cancer |
| Enavatuzumab | TWEAK Receptor | Cancer |
| Enlimomab | ICAM-1 (CD54) | Cancer |
| Enokizumab | IL9 | Asthma |
| Enoticumab | DLL4 | Cancer |
| Ensituximab | 5AC | Cancer |
| Epitumomab Cituxetan | Episialin | Cancer |
| Epratuzumab | CD22 | Cancer, SLE |
| Erlizumab | ITGB2 (CD18) | Heart Attack, Stroke, Traumatic Shock |
| Ertumaxomab | HER2/Neu, CD3 | Breast Cancer |
| Etaracizumab | Integrin Aᵥβ₃ | Melanoma, Prostate Cancer, Ovarian Cancer |
| Etrolizumab | Integrin A₇ B₇ | Inflammatory Bowel Disease |
| Evolocumab | PCSK9 | Hypocholesterolemia |
| Exbivirumab | Hepatitis B Surface Antigen | Hepatitis B |
| Fanolesomab | CD15 | Appendicitis (Diagnosis) |
| Faralimomab | Interferon Receptor | Cancer |
| Farletuzumab | Folate Receptor 1 | Ovarian Cancer |
| Fasinumab^{[51]} | HNGF | Cancer |
| Fbta05 | CD20 | Chronic Lymphocytic Leukaemia |
| Felvizumab | Respiratory Syncytial Virus | Respiratory Syncytial Virus Infection |
| Fezakinumab | IL-22 | Rheumatoid Arthritis, Psoriasis |
| Ficlatuzumab | HGF | Cancer |
| Figitumumab | IGF-1 Receptor | Adrenocortical Carcinoma, Non-Small Cell Lung Carcinoma |
| Flanvotumab | TYRP1 (Glycoprotein 75) | Melanoma |
| Fontolizumab | IFN-γ | Crohn's Disease |
| Foravirumab | Rabies Virus Glycoprotein | Rabies (Prophylaxis) |
| Fresolimumab | TGF-B | Idiopathic Pulmonary Fibrosis, Focal Segmental Glomerulosclerosis, Cancer |
| Fulranumab | NGF | Pain |
| Futuximab | EGFR | Cancer |
| Galiximab | CD80 | B Cell Lymphoma |
| Ganitumab | IGF-I | Cancer |
| Gantenerumab | Beta-Amyloid | Alzheimer's Disease |
| Gavilimomab | CD147 (Basigin) | Graft-Versus-Host Disease |
| Gemtuzumab Ozogamicin | CD33 | Acute Myelogenous Leukemia |
| Gevokizumab | IL-1β | Diabetes |
| Girentuximab | Carbonic Anhydrase 9 (CA-IX) | Clear Cell Renal Cell Carcinoma |
| Glembatumumab Vedotin | GPNMB | Melanoma, Breast Cancer |
| Golimumab | TNF-A | Rheumatoid Arthritis, Psoriatic Arthritis, Ankylosing Spondylitis |
| Gomiliximab | CD23 (Ige Receptor) | Allergic Asthma |
| Guselkumab | IL13 | Psoriasis |
| Ibritumomab Tiuxetan | CD20 | Non-Hodgkin's Lymphoma |
| Icrucumab | VEGFR-1 | Cancer |
| Igovomab | CA-125 | Ovarian Cancer (Diagnosis) |
| Imab362 | Cldn18.2 | Gastrointestinal Adenocarcinomas And Pancreatic Tumor |
| Imgatuzumab | EGFR | Cancer |
| Inclacumab | Selectin P | Cancer |
| Indatuximab Ravtansine | SDC 1 | Cancer |
| Infliximab | TNF-A | Rheumatoid Arthritis, Ankylosing Spondylitis, Psoriatic Arthritis, Psoriasis, Crohn's Disease, Ulcerative Colitis |
| Inolimomab | CD25 (A Chain Of IL-2 Receptor) | Graft-Versus-Host Disease |
| Inotuzumab Ozogamicin | CD22 | Cancer |
| Intetumumab | CD51 | Solid Tumors (Prostate Cancer, Melanoma) |
| Ipilimumab | CD152 | Melanoma |
| Iratumumab | CD30 (TNFRSF8) | Hodgkin's Lymphoma |
| Itolizumab | CD6 | Cancer |
| Ixekizumab | IL-17A | Autoimmune Diseases |
| Keliximab | CD4 | Chronic Asthma |
| Labetuzumab | CEA | Colorectal Cancer |
| Lambrolizumab | PDCD1 | Antineoplastic Agent |
| Lampalizumab | CFD | Cancer |
| Lebrikizumab | IL-13 | Asthma |
| Lemalesomab | NCA-90 (Granulocyte Antigen) | Diagnostic Agent |
| Lerdelimumab | TGF Beta 2 | Reduction Of Scarring After Glaucoma Surgery |
| Lexatumumab | TRAIL-R2 | Cancer |
| Libivirumab | Hepatitis B Surface Antigen | Hepatitis B |
| Ligelizumab | IGHE | Cancer |
| Lintuzumab | CD33 | Cancer |
| Lirilumab | KIR2D | Cancer |
| Lodelcizumab | PCSK9 | Hypercholesterolemia |
| Lorvotuzumab | CD56 | Cancer |
| Lucatumumab | CD40 | Multiple Myeloma, Non-Hodgkin's Lymphoma, Hodgkin's Lymphoma |
| Lumiliximab | CD23 | Chronic Lymphocytic Leukemia |
| Mapatumumab | TRAIL-R1 | Cancer |
| Margetuximab | Ch4d5 | Cancer |
| Matuzumab | EGFR | Colorectal, Lung And Stomach Cancer |
| Mavrilimumab | GMCSF Receptor A-Chain | Rheumatoid Arthritis |
| Mepolizumab | IL-5 | Asthma And White Blood Cell Diseases |
| Metelimumab | TGF Beta 1 | Systemic Scleroderma |
| Milatuzumab | CD74 | Multiple Myeloma And Other Hematological Malignancies |
| Minretumomab | TAG-72 | Cancer |
| Mitumomab | GD3 Ganglioside | Small Cell Lung Carcinoma |
| Mogamulizumab | CCR4 | Cancer |
| Morolimumab | Rhesus Factor | Cancer |
| Motavizumab | Respiratory Syncytial Virus | Respiratory Syncytial Virus (Prevention) |
| Moxetumomab Pasudotox | CD22 | Cancer |
| Muromonab-CD3 | CD3 | Prevention Of Organ Transplant Rejections |
| Nacolomab Tafenatox | C242 Antigen | Colorectal Cancer |
| Namilumab | CSF2 | Cancer |
| Naptumomab Estafenatox | 5T4 | Non-Small Cell Lung Carcinoma, Renal Cell Carcinoma |
| Narnatumab | RON | Cancer |
| Natalizumab | Integrin A4 | Multiple Sclerosis, Crohn's Disease |
| Nebacumab | Endotoxin | Sepsis |
| Necitumumab | EGFR | Non-Small Cell Lung Carcinoma |
| Nerelimomab | TNF-A | Cancer |
| Nesvacumab | Angiopoietin 2 | Cancer |
| Nimotuzumab | EGFR | Squamous Cell Carcinoma, Head And Neck Cancer, Nasopharyngeal Cancer, Glioma |
| Nivolumab | PD-1 | Cancer |
| Nofetumomab Merpentan | Undetermined | Cancer |
| Ocaratuzumab | CD20 | Cancer |
| Ocrelizumab | CD20 | Rheumatoid Arthritis, Lupus Erythematosus |
| Odulimomab | LFA-1 (CD11a) | Prevention Of Organ Transplant Rejections, Immunological Diseases |
| Ofatumumab | CD20 | Chronic Lymphocytic Leukemia |
| Olaratumab | PDGF-R A | Cancer |
| Olokizumab | IL6 | Cancer |
| Onartuzumab | Human Scatter Factor Receptor Kinase | Cancer |
| Ontuxizumab | TEM1 | Cancer |
| Oportuzumab Monatox | Epcam | Cancer |
| Oregovomab | CA-125 | Ovarian Cancer |
| Orticumab | Oxldl | Cancer |
| Otlertuzumab | CD37 | Cancer |
| Oxelumab | OX-40 | Asthma |
| Ozanezumab | NOGO-A | ALS And Multiple Sclerosis |
| Ozoralizumab | TNF-A | Inflammation |
| Pagibaximab | Lipoteichoic Acid | Sepsis (*Staphylococcus*) |
| Palivizumab | F Protein Of Respiratory Syncytial Virus | Respiratory Syncytial Virus (Prevention) |
| Panitumumab | EGFR | Colorectal Cancer |
| Pankomab | Tumor Specific Glycosylation Of MUC1 | Ovarian Cancer |
| Panobacumab | Pseudomonas Aeruginosa | *Pseudomonas Aeruginosa* Infection |
| Parsatuzumab | EGFL7 | Cancer |
| Pascolizumab | IL-4 | Asthma |
| Pateclizumab | LTA | TNF |
| Patritumab | HER3 | Cancer |
| Pembrolizumab | PD-1 | Cancer |
| Pemtumomab | MUC1 | Cancer |
| Perakizumab | IL17A | Arthritis |
| Pertuzumab | HER2/Neu | Cancer |
| Pexelizumab | C5 | Reduction Of Side-Effects Of Cardiac Surgery |
| Pidilizumab | PD-1 | Cancer And Infectious Diseases |
| Pinatuzumab Vedotin | CD22 | Cancer |
| Pintumomab | Adenocarcinoma Antigen | Adenocarcinoma |
| Placulumab | Human TNF | Cancer |
| Polatuzumab Vedotin | CD79B | Cancer |
| Ponezumab | Human Beta-Amyloid | Alzheimer's Disease |
| Pritoxaximab | *E. Coli* Shiga Toxin Type-1 | Cancer |
| Pritumumab | Vimentin | Brain Cancer |
| Pro 140 | Ccr5 | HIV Infection |
| Quilizumab | IGHE | Cancer |
| Racotumomab | N-Glycolylneuraminic Acid | Cancer |
| Radretumab | Fibronectin Extra Domain-B | Cancer |
| Rafivirumab | Rabies Virus Glycoprotein | Rabies (Prophylaxis) |
| Ramucirumab | VEGFR2 | Solid Tumors |
| Ranibizumab | VEGF-A | Macular Degeneration (Wet Form) |
| Raxibacumab | Anthrax Toxin, Protective Antigen | Anthrax (Prophylaxis And Treatment) |
| Regavirumab | Cytomegalovirus Glycoprotein B | Cytomegalovirus Infection |
| Reslizumab | IL-5 | Inflammations Of The Airways, Skin And Gastrointestinal Tract |
| Rilotumumab | HGF | Solid Tumors |
| Rituximab | CD20 | Lymphomas, Leukemias, Some Autoimmune Disorders |
| Robatumumab | IGF-1 Receptor | Cancer |
| Roledumab | RHD | Cancer |
| Romosozumab | Sclerostin | Osteoporosis |
| Rontalizumab | IFN-α | Systemic Lupus Erythematosus |
| Rovelizumab | CD11, CD18 | Haemorrhagic Shock |
| Ruplizumab | CD154 (CD40L) | Rheumatic Diseases |
| Samalizumab | CD200 | Cancer |
| Sarilumab | IL6 | Rheumatoid Arthritis, Ankylosing Spondylitis |
| Satumomab Pendetide | TAG-72 | Cancer |
| Secukinumab | IL-17A | Uveitis, Rheumatoid Arthritis Psoriasis |
| Seribantumab | ERBB3 | Cancer |
| Setoxaximab | *E. Coli* Shiga Toxin Type-1 | Cancer |
| Sevirumab | Cytomegalovirus | Cytomegalovirus Infection |
| Sgn-CD19a | CD19 | Acute Lymphoblastic Leukemia And B Cell Non-Hodgkin Lymphoma |
| Sgn-CD33a | CD33 | Acute Myeloid Leukemia |
| Sibrotuzumab | FAP | Cancer |
| Sifalimumab | IFN-A | SLE, Dermatomyositis, Polymyositis |
| Siltuximab | IL-6 | Cancer |
| Simtuzumab | LOXL2 | Fibrosis |
| Siplizumab | CD2 | Psoriasis, Graft-Versus-Host Disease (Prevention) |
| Sirukumab | IL-6 | Rheumatoid Arthritis |
| Solanezumab | Beta-Amyloid | Alzheimer's Disease |
| Solitomab | Epcam | Cancer |
| Sonepcizumab | Sphingosine-1-Phosphate | Choroidal And Retinal Neovascularization |
| Sontuzumab | Episialin | Cancer |
| Stamulumab | Myostatin | Muscular Dystrophy |
| Sulesomab | NCA-90 (Granulocyte Antigen) | Osteomyelitis |
| Suvizumab | HIV-1 | Viral Infections |
| Tabalumab | BAFF | B Cell Cancers |
| Tacatuzumab Tetraxetan | Alpha-Fetoprotein | Cancer |
| Tadocizumab | Integrin AIIBβ3 | Percutaneous Coronary Intervention |
| Tanezumab | NGF | Pain |
| Taplitumomab Paptox | CD19 | Cancer |
| Tefibazumab | Clumping Factor A | *Staphylococcus Aureus* Infection |
| Telimomab | Undetermined | Cancer |
| Tenatumomab | Tenascin C | Cancer |
| Teneliximab | CD40 | Cancer |
| Teprotumumab | CD221 | Hematologic Tumors |
| Ticilimumab | CTLA-4 | Cancer |
| Tigatuzumab | TRAIL-R2 | Cancer |
| Tildrakizumab | IL23 | Immunologically Mediated Inflammatory Disorders |
| Tnx-650 | Il-13 | Hodgkin's Lymphoma |
| Tocilizumab | IL-6 Receptor | Rheumatoid Arthritis |
| Toralizumab | CD154 (CD40L) | Rheumatoid Arthritis, Lupus Nephritis |
| Tositumomab | CD20 | Follicular Lymphoma |
| Tovetumab | CD140a | Cancer |
| Tralokinumab | IL-13 | Asthma |
| Trastuzumab | HER2/Neu | Breast Cancer |
| Trbs07 | Gd2 | Melanoma |
| Tremelimumab | CTLA-4 | Cancer |
| Tucotuzumab Celmoleukin | Epcam | Cancer |
| Tuvirumab | Hepatitis B Virus | Chronic Hepatitis B |
| Ublituximab | MS4A1 | Cancer |
| Urelumab | 4-1BB | Cancer |
| Urtoxazumab | Escherichia Coli | Diarrhoea Caused By *E*. *Coli* |
| Ustekinumab | IL-12, IL-23 | Multiple Sclerosis, Psoriasis, Psoriatic Arthritis |
| Vantictumab | Frizzled Receptor | Cancer |
| Vapaliximab | AOC3 (VAP-1) | Cancer |
| Vatelizumab | ITGA2 | Cancer |
| Vedolizumab | Integrin A4β7 | Crohn's Disease, Ulcerative Colitis |
| Veltuzumab | CD20 | Non-Hodgkin's Lymphoma |
| Vepalimomab | AOC3 (VAP-1) | Inflammation |
| Vesencumab | NRP1 | Cancer |
| Volociximab | Integrin A5β1 | Solid Tumors |
| Vorsetuzumab | CD70 | Cancer |
| Votumumab | Tumor Antigen CTAA16.88 | Colorectal Tumors |
| Zalutumumab | EGFR | Squamous Cell Carcinoma Of The Head And Neck |
| Zatuximab | HER1 | Cancer |
| Ziralimumab | CD 147 | Cancer |
| Zolimomab Aritox | CD5 | Systemic Lupus Erythematosus, Graft-Versus-Host Disease |

| **Table 2 Hormones / Factors** |
|---|
| Alpha-Glactosidase A |
| Alpha-L-Iduronidase |
| Dornase Alfa |
| Erythropoietin |
| Factor VIII |
| Follicle-Stimulating Hormone |
| Glucocerebrosidase |
| Granulocyte Colony-Stimulating Factor (G-CSF) |
| Growth Hormone |
| Insulin |
| Insulin-Like Growth Factor 1 (IGF-1) |
| Interferon-B-1α |
| Interferon-B-1β |
| N-Acetylgalactosamine-4-Sulfatase |
| Tissue Plasminogen Activator (TPA) |

### IV. Formulation of the Pharmaceutical Compositions of the Present Invention

The pharmaceutical compositions of the present invention comprise an aqueous carrier and are then lyophilized. The pharmaceutical compositions of the present invention subsequent to such lyophilization is referred to herein as a "lyophilisate."

The lyophilized formulations of the pharmaceutical compositions of the present invention comprise a suitable sterile aqueous carrier, a high concentration (as defined above) of the protein biomolecule, a buffer, and a stabilizing compound of the present invention. Optionally, such lyophilized formulations of the pharmaceutical compositions of the present invention may contain additional components, for example, a pharmaceutically acceptable, non-toxic excipient, buffer or detergent. The pharmaceutical compositions of the present invention preferably lack sugar, or are substantially free of sugar.

Examples of suitable sterile aqueous carriers which may be employed in the pharmaceutical compositions of the present invention include water, saline, phosphate buffered saline, ethanol, dextrose solutions, and water/polyol solutions (such as glycerol, propylene glycol, polyethylene glycol, and the like).

The buffer is capable of buffering the liquid to a pH of 6.

The buffer is used at a molarity of 25 mM. The buffer is histidine / histidine HCl. Histidine can be in the form of L-histidine, D-histidine, or a mixture thereof, but L-histidine is the most preferable. Histidine can be also in the form of a hydrate, or a hydrochloride (e.g. a monohydrochloride or a dihydrochloride). The purity of the histidine should be at least 98%, preferably at least 99%, and most preferably at least 99.5%.

The concentration of the stabilizing compound(s) that is/are included in the composition of the present invention is according to claim 1, up to 6% w/v.

Polysorbate-80 ("PS-80") is a non-ionic surfactant and emulsifier of the present invention, however, other suitable non-ionic surfactants and emulsifiers (*e.g*., Tween-20^{®}, Tween- 80^{®}, Polaxamers, sodium dodecyl sulfate, *etc.*) may be additionally employed.

The lyophilized formulations according to the invention comprise: (2)(a) 75 mg/mL of a protein biomolecule, 25 mM histidine/histidine-HCl, 5% w/v arginine, 0.02% w/v PS-80 and a pH of 6; or (2)(b) 100 mg/mL of a protein biomolecule, 25 mM histidine/histidine-HCl, 4% w/v alanine, 2% w/v arginine, 0.02% w/v PS-80 and a pH of 6.

The liquid formulations are lyophilized to further stabilize the protein biomolecule. Any suitable lyophilization apparatus and regimen may be employed, however, it is preferred to accomplish such lyophilization as shown in Table 3, Table 5 or Table 11.

Particularly subsequent to reconstitution after such lyophilization, liquid formulations of the pharmaceutical compositions of the present invention may additionally contain non-aqueous carriers, such as mineral oil or vegetable oil (*e.g.,* olive oil, corn oil, peanut oil, cottonseed oil, and sesame oil), carboxymethyl cellulose colloidal solutions, tragacanth gum and injectable organic esters, such as ethyl oleate.

The invention provides compositions for use in the treatment, prophylaxis, and amelioration of a disease or condition or one or more symptoms thereof by administrating to a subject of an effective amount of the formulations of the invention, either as initially formulated or subsequent to reconstitution of a lyophilisate.

Various delivery systems are known and can be used to administer such compositions, including, but not limited to, parenteral administration (e.g., intradermal, intramuscular, intraperitoneal, intravenous and subcutaneous), epidural administration, topical administration, pulmonary administration, and mucosal administration (e.g., intranasal and oral mutes). In a specific embodiment, formulations of the present invention are administered intramuscularly, intravenously, or subcutaneously. The formulations may be administered by any convenient mute, for example by infusion or bolus injection, by absorption through epithelial or mucocutaneous linings (e.g., oral mucosa, rectal and intestinal mucosa, etc.) and may be administered together with other biologically active agents. Administration can be systemic or local. In addition, pulmonary administration can be employed, *e.g.,* by use of an inhaler or nebulizer.

The invention also provides that the initially formulated liquid pharmaceutical composition may be packaged in a hermetically sealed container such as an ampoule, vial, cartridge, syringe or sachette indicating the quantity of the protein biomolecule contained therein. Said initially formulated liquid pharmaceutical compositions are lyophilized while within such ampoules or sachettes, and the ampoule or sachette indicates the amount of carrier to be added in order to reconstitute the lyophilisate to contain the desired high concentration of the protein biomolecule.

The amount of the lyophilized formulations of the present invention which will be effective for therapeutic or prophylactic use.

The precise dose to be employed in the formulation will also depend on the route of administration, the disease or condition to be treated, the particular protein biomolecule of the pharmaceutical composition, and should be decided according to the judgment of the practitioner and each subject's circumstances. Exemplary doses include 30 mg/kg or less, 15 mg/kg or less, 5 mg/kg or less, 3 mg/kg or less, 1 mg/kg or less or 0.5 mg/kg or less.

### Examples

The following examples illustrate working compositions and compositions according to the present invention and their properties. The examples are intended to illustrate, but in no way limit, the scope of the invention.

### Example 1

### Materials & Methods

**Lyophilization** - 1.1 mL aliquots of a pharmaceutical composition were introduced into 3 cc glass vials. The vials were stoppered with 13 mm single vent lyophilization stoppers. The vials were then lyophilized using a lyophilization cycle, as described in Table 3.

| **Table 3 Lyophilization Parameters** | |
|---|---|
| Loading | 20 °C |
| Freezing | -5 °C at 0.3 °C/min |
| Annealing | -16 °C at 0.5 °C/min |
| Freezing | -40 °C at 0.5 °C/min |
| Primary Drying | -35 °C at 0.1 °C/min |
| Secondary Drying | 40 °C at 0.3 ° C/min |
| Unloading | 5 °C |

The end point of lyophilization was determined using a Pirani vacuum gauge (see, *e.g.,* Patel, S.M. et al. (2009) "Determination of End Point of Primary Drying in Freeze-Drying Process Control," AAPS Pharm. Sci. Tech. 11(1):73-84). Such a gauge works on the principle of measuring the thermal conductivity of the gas in the drying chamber (Nail, S.L. et al. (1992) "Methodology For In-Process Determination Of Residual Water In Freeze-Dried Products," Dev. Biol. Stand. 74:137-151; Biol. Prod. Freeze-Drying Formulation). After completion of the lyophilization cycles, vials were vacuum stoppered and removed from the lyophilizer. The vials were then capped with West 13 mm aluminum Flip-Off overseals.

**High Performance Size-Exclusion Chromatography (HPSEC)** - HPSEC samples were diluted in 10 mg/mL phosphate buffered saline prior to HPSEC. The samples were injected onto a TSKgel G3000SWXL column, eluted isocratically with phosphate buffer containing sodium sulfate and sodium azide. The eluted protein is detected using UV absorbance at 280 nm and the results are reported as the area percent of the product monomer peak. Peaks eluting earlier than the monomer are recorded as percent aggregate and peaks eluting after the monomer are recorded as percent fragment/other.

**Reconstitution Procedure** - Prior to use, and generally within 6 hours prior to use, sterile water is injected into the lyophilization vial, which is then gently swirled to effect reconstitution with minimal foaming. Two reconstitution procedures were used for reconstitution: Procedure A - a 1 minute swirl followed by a 5 minute hold method until all the cake is completely dissolved in solution and Procedure B - a 1 minute hold followed by a 1 minute swirl until all the cake is completely dissolved in solution.

### Example 2

### Impact of Variation of Amino Acid to Sugar Ratio on Reconstitution Time and Protein Aggregation of Pharmaceutical Compositions

In order to investigate the effect of varying the ratio of amino acid to sugar concentrations in pharmaceutical compositions on the preparation, stability and storage of such compositions, a pharmaceutical composition containing an exemplary protein biomolecule (a human IgG1 monoclonal antibody) was incubated in formulations containing different amino acids and at differing amino acid to sugar ratios. More specifically, the pharmaceutical composition was formulated at 100 mg/mL in 25mM histidine/histidine-HCl, 0.02% (w/v) polysorbate-80 (PS-80), pH 6 buffer with arginine-HCl, lysine-HCl, proline, alanine or glycine at amino acid to sugar ratios as shown in Table 4 and the preparations were evaluated for their effect on reconstitution times of the lyophilized formulations.

| **Table 4 Composition of Amino Acid Formulations** | | | | | | |
|---|---|---|---|---|---|---|
| **Description** | **Protein (mg/mL)** | **Amino Acid (mg/mL)** | | **Sucrose (mg/mL)** | | **Amino Acid : Sucrose Ratio** |
| Amino Acid Formulation | 100 | 50 | | 0 | | 5:0 |
| | | 40 | | 10 | (1 % (w/v)) | 4:1 |
| | | 25 | | 50 | (5% (w/v)) | 2:1 |
| Control | 100 | 0 | | 100 | (10% (w/v)) | N/A |

The formulations were lyophilized according to the process in Table 5.

| **Table 5 Lyophilization Process** | |
|---|---|
| Loading | 20 °C |
| Freezing | -5 °C at 0.3 °C/min |
| Annealing | No Annealing |
| Freezing | -40 °C at 0.1 °C/min |
| Primary Drying | -35 °C at 0.1 °C/min |
| Secondary Drying | 40 °C at 0.3 °C/min |
| Unloading | 5 °C |

**Figure 1** summarizes the aggregation and reconstitution time results. All formulations of the above-described pharmaceutical composition containing the exemplary protein biomolecule that contained lysine-HCl had high, and in some cases, unacceptably high reconstitution times. Formulations of the pharmaceutical composition that contained arginine, lysine-HCl or proline did not show an in-process increase in aggregation. In contrast, formulations of the pharmaceutical composition that contained alanine and glycine showed an increase in in-process aggregate level, but the addition of amorphous content (sucrose) minimized or prevented this increase, depending on the ratio employed. The results show that arginine, lysine and proline could substitute for sucrose without affecting aggregation.

The lyophilized formulations were also subjected to X-ray Powder Diffraction (XRPD) in order to determine the crystallinity of the lyophilisate. The results, as well as reconstitution times are shown in Table 6 (Reconstitution Time (RC) in minutes; n=2; XRPD, n=1; A, Amorphous; M, Mixture of Amorphous and Crystalline).

| **Table 6 Impact of Amino Acid to Sucrose Ratio on Reconstitution Time and XRPD** | | | | | | | | | | |
|---|---|---|---|---|---|---|---|---|---|---|
| Amino Acid to Sugar Ratio | Arginine-HCl | | Lysine-HCl | | Alanine | | Proline | | Glycine | |
| | RC | XRPD | RC | XRPD | RC | XRPD | RC | XRPD | RC | XRPD |
| 5:0 | <9 | (A) | <50 | (A) | <5 | (A) | <30 | (A) | <5 | M |
| 4:1 | <29 | (A) | <50 | (A) | <5 | (A) | <30 | (A) | <5 | M |
| 1:2 | <29 | (A) | <50 | (A) | <24 | (A) | <30 | (A) | <24 | (A) |
| Control | <27 (A) | | | | | | | | | |

In summary, formulations of the pharmaceutical composition containing the exemplary protein biomolecule that contained arginine alone showed a significantly lower reconstitution time compared to the sucrose only formulation. The addition of even 1% (w/v) of sucrose to the arginine formulations increased the reconstitution time. All formulations with arginine were amorphous as measured by XRPD. Formulations of the pharmaceutical composition that contained alanine or glycine showed rapid reconstitution in the absence of sucrose, or in the presence of 1% (w/v) sucrose, but the addition of 5% (w/v) and higher sucrose concentrations increased reconstitution times. Formulations of the pharmaceutical composition that contained alanine or glycine and 0-1% (w/v) sucrose showed a mixture of amorphous and crystalline product by XRPD, while addition of high sucrose to these formulations resulted in an amorphous matrix as determined by XRPD. Formulations of the pharmaceutical composition that contained lysine or proline were difficult to reconstitute and hence had longer reconstitution time. All the lysine- and proline-containing formulations were, however, amorphous as determined by XRPD. The results show that the presence of arginine, alanine or glycine could significantly reduce reconstitution time.

### Example 3

### Optimizing Sugar to Amino Acid Ratios in High Concentration Protein Formulations

The data presented in Example 2 indicates that both alanine and glycine have a tendency to crystallize when lyophilized alone or in the presence of low amounts of sugar. The following study was carried out to optimize ratios of sugar to amino acid (using alanine or glycine) to obtain amorphous lyophilized cakes with acceptable stability and short reconstitution times. Amino acid/sucrose formulations with various amino acid to sugar ratios were prepared for both alanine and glycine as shown in Table 7. The formulations were lyophilized according to the process shown in Table 5 with addition of annealing at -16 °C for 300 minutes. The lyophilisates were subjected to XRPD, and were then reconstituted. Reconstitution times, percent aggregate increase over pre lyophilization solutions, and osmolality were measured.

| **Table 7** | | |
|---|---|---|
| **Amino Acid (mg/mL)** | **Sugar (mg/mL)** | **Amino Acid to Sugar Ratio** |
| 50 | 0 | 5:0 |
| 40 | 10 | 4:1 |
| 40 | 20 | 2:1 |
| 40 | 30 | 4:3 |
| 40 | 40 | 1:1 |
| 25 | 50 | 1:2 |

Table 8 summarizes the effect of amino acid to sugar ratios on reconstitution time, and increase in aggregate post-lyophilization. Results indicate that increasing sugar in the formulations prevents aggregate formation during the lyophilization process but increases reconstitution time. The results provide a guide to determine an acceptable balance between aggregation and reconstitution time, by adjusting the amino acid to sugar ratio.

| **Table 8 Study Results** | | | | | |
|---|---|---|---|---|---|
| **Amino Acid** | **Amino Acid to Sucrose Ratio** | **% Aggregate Increase (Post-Lyophilization)** | | **Reconstitution Time (n=2) in min** | |
| **Alanine** | 5:0 | | 0.8 | 4 | |
| | 4:1 | | 0.4 | 4 | |
| | 2:1 | | 0.1 | 18 | |
| | 4:3 | | 0.2 | 14 | |
| | 1:1 | | 0.2 | 18 | |
| | 1:2 | | 0.1 | 17 | |
| **Glycine** | 5:0 | | 1.5 | 5 | |
| | 4:1 | | 0.6 | 5 | |
| | 2:1 | | 0.1 | 13 | |
| | 4:3 | | 0.2 | 22 | |
| | 1:1 | | 0 | 24 | |
| | 1:2 | | 0.6 | 24 | |

### Example 4

### Evaluation of High Concentration Protein/Amino Acid Formulations

As observed in Example 2, high concentration protein formulations with arginine-HCl remained amorphous during lyophilization, indicating that arginine-HCl can act as a cryoprotectant and as a lyoprotectant. Additionally, the arginine alone protein formulation exhibited a reduced reconstitution time. Because of these characteristics, arginine was evaluated in combination with alanine and/or glycine in a series of high concentration formulations of the above-described pharmaceutical composition containing the exemplary protein biomolecule. In this study, the impact of protein concentration and amino acid ratio on reconstitution time was evaluated. The formulations evaluated are shown with a check mark in Table 9 (N/A, not applicable). The formulations were lyophilized according to the process shown in Table 3. The lyophilisates were subjected to XRPD, and were then reconstituted. Reconstitution times were measured.

| **Table 9 Study Design** | | | | | | | |
|---|---|---|---|---|---|---|---|
| **Amino Acid (% (w/v))** | | | | **Protein Concentration (mg/mL)** | | | |
| **Alanine** | **Glycine** | **Arginine** | | **50** | **75** | **90** | **100** |
| N/A | | | 2 | N/A | | | ✔ |
| | | | 3 | | | | ✔ |
| | | | 3.5 | ✔ | N/A | | ✔ |
| | | | 5 | ✔ | ✔ | ✔ | ✔ |
| 2 | N/A | | 2 | ✔ | ✔ | ✔ | ✔ |
| 4 | | | 2 | ✔ | ✔ | ✔ | ✔ |
| N/A | 2 | | 2 | ✔ | ✔ | ✔ | ✔ |
| | 4 | | 2 | ✔ | ✔ | ✔ | ✔ |

The reconstitution times of the various formulations are shown in Figure 2. These data show that protein concentration had an effect on the reconstitution time. As the protein concentration increased, reconstitution time increased. Also, the extent of increase in reconstitution time was affected by the type and quantity of the amino acid(s) present in the formulations. Both 3.5% (w/v) arginine and 5% (w/v) arginine had a similar impact on reconstitution time.

Formulations containing combinations of 4% glycine (w/v) or 4% alanine (w/v) with 2% arginine (w/v) exhibited reconstitution times reduced to about 10 minutes for the 100 mg lyophilized formulations (*i.e*., approximately ⅓ to ½ the reconstitution time observed using other combinations of solutes). Also, the extent of reduction in reconstitution time was dependent on amino acid ratio. For example, 2:1 glycine:arginine or alanine: arginine was more effective in reducing reconstitution time than 1:1 glycine:arginine or alanine:arginine.

The XRPD results demonstrated that all of the formulations except for 2:1 glycine:arginine were amorphous (Table 10; A, Amorphous; M, Mixture of Amorphous and Crystalline; N/A, not applicable).

| **Table 10 XRPD Results** | | | | | |
|---|---|---|---|---|---|
| **Amino Acid (% (w/v))** | | | | **Nominal Protein Concentration (mg/mL)** | |
| **Alanine** | **Glycine** | **Arginine** | | **50** | **100** |
| N/A | | | 2 | N/A | A |
| | | | 3.5 | A | N/A |
| | | | 5 | A | N/A |
| 2 | N/A | | 2 | A | A |
| 4 | | | 2 | A | A |
| N/A | 2 | | 2 | A | A |
| | 4 | | 2 | M | M |

Based on the results in Table 10 and in Figure 2, the following lyophilized formulations of the pharmaceutical composition were evaluated for stability at 5 °C, 25 °C 60% relative humidity and 40 °C 75% relative humidity:
(1) 75 mg/mL, 25 mM histidine/histidine-HCl, 3.5% arginine (w/v), and 0.02% PS-80 (w/v), pH 6;
(2) 75 mg/mL, 25 mM histidine/histidine-HCl, 5% arginine (w/v), and 0.02% PS-80 (w/v), pH 6;
(3) 100 mg/mL, 25 mM histidine/histidine-HCl, 4% alanine (w/v), 2% arginine (w/v), and 0.02% PS-80 (w/v), pH 6;
(4) 100 mg/mL, 25 mM histidine/histidine-HCl, 4% glycine (w/v), 2% arginine (w/v), and 0.02% PS-80 (w/v), pH 6;

Prior to lyophilization, 1.1 mL of the above-described four formulations were subjected to uncontrolled 1x freeze/thaw (F/T) in 3 cc vials (freezing at -80 °C and thawing at room temperature). HPSEC was monitored pre- and post-thaw to study the impact of the freeze/thaw cycle. No significant change in purity was observed in the freeze/thaw cycle.

Figures 3A and 3B show the stability of the lyophilisates at 40 °C and 25 °C, respectively. The stability was monitored for 6 months at 25 °C and for 3 months at 40 °C. The rates of purity loss at both 25 °C and 40 °C were similar to sucrose containing formulations. Post 3 months at 40 °C, samples of the lyophilized pharmaceutical composition formulations were submitted to XRPD analysis. Based on XRPD analysis, all of the formulations except those containing glycine were amorphous. Glycine-containing formulations showed a mixture of amorphous and crystalline component, which is consistent with initial (T-0) observations. The stability was also evaluated at 5 °C for 22 months and showed no change in purity.

Ten vials of each formulation (post-lyophilization) were reconstituted and the reconstitution times were measured. The results are shown in Figure 4. The average reconstitution times of all formulations were less than 15 minutes. The 100 mg/mL formulation of the pharmaceutical composition containing the exemplary protein biomolecule containing a combination of glycine and arginine was the most effective in reducing the reconstitution time followed by the combination of alanine and arginine. For the 75 mg/mL formulation of the pharmaceutical composition containing the exemplary protein biomolecule, both the 3.5% and 5% arginine (w/v) formulations provided acceptable reconstitution times.

### Example 5

### Evaluation of the Impact of Molecule Type on Reconstitution Time

In order to understand the impact of molecule type on reconstitution time and to demonstrate the generality of the present invention with respect to any protein biomolecule, pharmaceutical compositions were prepared using alternative protein biomolecules. Specifically, pharmaceutical compositions were prepared employing a Tenascin-3-Human Serum Albumin (Tn3-HSA) fusion protein (see, *e.g.,* PCT Publication No. WO 2013/055745) or a humanized IgG4 monoclonal antibody in lieu of the human IgG1 monoclonal antibody of the above-described pharmaceutical compositions. The employed formulations were the four lead formulations noted in Example 4 (reiterated below):
(1) 75 mg/mL, 25 mM histidine/histidine-HCl, 3.5% arginine, 0.02% PS-80, pH 6;
(2) 75 mg/mL, 25 mM histidine/histidine-HCl, 5% arginine, 0.02% PS-80, pH 6;
(3) 100 mg/mL, 25 mM histidine/histidine-HCl, 4% alanine, 2% arginine, 0.02% PS-80, pH 6;
(4) 100 mg/mL, 25 mM histidine/histidine-HCl, 4% glycine, 2% arginine, 0.02% PS-80, pH 6;

The additional pharmaceutical compositions were formulated as indicated, and lyophilized according to the process in Table 11.

| **Table 11 Lyophilization Process** | |
|---|---|
| Loading | 20 °C |
| Freezing | -5 °C at 0.3 °C/min |
| Annealing | -16 °C at 0.5 °C/min |
| Freezing | -40 °C at 0.5 °C/min |
| Primary Drying | -35 °C at 0.1 °C/min |
| Secondary Drying | 40 °C at 0.3 ° C/min |
| Unloading | 5 °C |

Lyophilisates samples are submitted for stability evaluations at various temperatures. Percent aggregation of the samples is evaluated by HPSEC. Following lyophilization, samples were reconstituted. Formulations were reconstituted using one of two alternative procedures (Procedure A was employed with the human IgG1 monoclonal antibody, and Procedure B was employed with the Tn3-HSA fusion protein and the humanized IgG4 monoclonal antibody). The reconstitution times for the IgG1 antibody are shown in Figure 5A. The reconstitution times for the Tn3-HSA fusion protein are shown in Figure 5B. The reconstitution time for the IgG4 antibody are shown in Figure 5C. The reconstitution times for the three molecules were significantly lower, compared to sucrose only formulations and were all 15 minutes or less.

## Claims

1. A lyophilisate of a pharmaceutical composition comprising a protein biomolecule as an active agent or component thereof, wherein said composition comprises:
(1) an aqueous carrier; and
(2)(a) 75 mg/mL of a protein biomolecule, 25 mM histidine/ histidine-HCl as a buffer, 5% arginine w/v as a stabilizing compound wherein the total concentration of the stabilizing compound is up to 6% w/v selected from the group consisting of arginine, alanine, glycine, lycine, or proline or salt thereof, 0.02% PS-80 w/v, and a pH of 6; or
(2)(b) 100 mg/mL of a protein biomolecule, 25 mM histidine/ histidine-HCl as a buffer, 4% alanine w/v and 2% arginine w/v as stabilizing compounds wherein the total concentration of the stabilizing compound is up to 6% w/v selected from the group consisting of arginine, alanine, glycine, lycine, or proline or salt thereof, 0.02% PS-80 w/v, and a pH of 6;
wherein said protein biomolecule is an antibody or an antibody-based immunotherapeutic.

2. The pharmaceutical composition of claim 1, wherein the composition completely lacks a sugar stabilizing compound.

3. The pharmaceutical composition of any one of claims 1-2, wherein the composition comprises at least two protein biomolecules.

4. The pharmaceutical composition of any one of claims 1-3, wherein the composition comprises at least two stabilizing compounds.

5. An ampoule, vial, cartridge, syringe or sachette that contains the pharmaceutical composition of any one of claims 1-4.

6. The pharmaceutical composition of any one of claims 1-4 for use as a medicament.

## Patentansprüche

1. Lyophilisat einer pharmazeutischen Zusammensetzung, umfassend ein Proteinbiomolekül als Wirkstoff oder -komponente davon, wobei die Zusammensetzung Folgendes umfasst:
(1) einen wässrigen Träger; und
(2) (a) 75 mg/ml eines Proteinbiomoleküls, 25 mM Histidin/Histidin-HCl als Puffer, 5% Gew./Vol. Arginin als stabilisierende Verbindung, wobei die Gesamtkonzentration der stabilisierenden Verbindung bis zu 6% Gew./Vol. beträgt, ausgewählt aus der Gruppe bestehend aus Arginin, Alanin, Glycin, Lycin oder Prolin oder Salz davon, 0,02% Gew./Vol. PS-80 und einen pH von 6; oder
(2) (b) 100 mg/ml eines Proteinbiomoleküls, 25 mM Histidin/Histidin-HCl als Puffer, 4% Gew./Vol. Alanin und 2% Gew./Vol. Arginin als stabilisierende Verbindungen, wobei die Gesamtkonzentration der stabilisierenden Verbindung bis zu 6% Gew./Vol. beträgt, ausgewählt aus der Gruppe bestehend aus Arginin, Alanin, Glycin, Lycin oder Prolin oder Salz davon, 0,02% Gew./Vol. PS-80 und einen pH von 6;
wobei es sich bei dem Proteinbiomolekül um einen Antikörper oder ein Immuntherapeutikum auf Antikörperbasis handelt.

2. Pharmazeutische Zusammensetzung nach Anspruch 1, wobei der Zusammensetzung eine stabilisierende Zuckerverbindung komplett fehlt.

3. Pharmazeutische Zusammensetzung nach einem der Ansprüche 1-2, wobei die Zusammensetzung wenigstens zwei Proteinbiomoleküle umfasst.

4. Pharmazeutische Zusammensetzung nach einem der Ansprüche 1-3, wobei die Zusammensetzung wenigstens zwei stabilisierende Verbindungen umfasst.

5. Ampulle, Fläschchen, Kartusche, Spritze oder Tütchen, die bzw. das die pharmazeutische Zusammensetzung nach einem der Ansprüche 1-4 enthält.

6. Pharmazeutische Zusammensetzung nach einem der Ansprüche 1-4 zur Verwendung als Arzneimittel.

## Revendications

1. Lyophilisat d'une composition pharmaceutique comprenant une biomolécule de protéine en tant qu'agent ou composant actif correspondant, ladite composition comprenant :
(1) un support aqueux ; et
(2) (a) 75 mg/mL d'une biomolécule de protéine, 25 mM d'histidine/histidine-HCl en tant que tampon,
5 % d'arginine p/v en tant que composé stabilisant, la concentration totale du composé stabilisant étant jusqu'à 6 % p/v, choisi dans le groupe constitué par l'arginine, l'alanine, la glycine, la lycine, ou la proline ou un sel correspondant, 0,02 % de PS-80 p/v, et un pH de 6 ; ou
(2) (b) 100 mg/mL d'une biomolécule de protéine, 25 mM d'histidine/histidine-HCl en tant que tampon,
4 % d'alanine p/v et 2 % d'arginine p/v en tant que composés stabilisants, la concentration totale du composé stabilisant étant jusqu'à 6 % p/v, choisis dans le groupe constitué par l'arginine, l'alanine, la glycine, la lycine, ou la proline ou un sel correspondant, 0,02 % de PS-80 p/v, et un pH de 6 ;
ladite biomolécule de protéine étant un anticorps ou une substance immunothérapeutique à base d'anticorps.

2. Composition pharmaceutique selon la revendication 1, la composition faisant complètement défaut d'un composé stabilisant de type sucre.

3. Composition pharmaceutique selon l'une quelconque des revendications 1 et 2, la composition comprenant au moins deux biomolécules de protéine.

4. Composition pharmaceutique selon l'une quelconque des revendications 1 à 3, la composition comprenant au moins deux composés stabilisants.

5. Ampoule, flacon, cartouche, seringue ou sachet qui contient la composition pharmaceutique selon l'une quelconque des revendications 1 à 4.

6. Composition pharmaceutique selon l'une quelconque des revendications 1 à 4 pour une utilisation en tant que médicament.
